# EUROPEAN PATENT SPECIFICATION

(11) **EP 3 166 540 B1**
(45) Date of publication and mention of the grant of the patent: **19.06.2019**
(21) Application number: 14739390.4
(22) Date of filing: 09.07.2014
(51) Int. Cl.: A61F 2/38, A61F 2/36, A61B 17/15, A61B 17/17, A61F 2/30

(54) **A SURGICAL JOINT IMPLANT**
CHIRURGISCHES GELENKIMPLANTAT
IMPLANT D'ARTICULATION CHIRURGICAL

(43) Date of publication of application: 17.05.2017
(73) Proprietor: Episurf IP-Management AB, 114 49 Stockholm (SE)
(72) Inventor: BAKE, Nina, 181 30 Lidingö (SE); LILLIESTRÅLE, Richard, 113 37 Stockholm (SE)
(74) Representative: Theander, Anna Katarina Henrietta
(86) International application number: PCT/EP2014/064760
(87) International publication number: WO 2016/004992

(56) References cited:
- EP-A2- 1 864 629
- US-A- 5 716 360
- US-A1- 2003 144 741
- US-A1- 2013 165 939
- US-A1- 2013 211 410

## Description

### Introduction

The present invention relates to a surgical joint implant. The implant according to the invention is intended for repair of the surface of a joint of a human or animal.

It is intended that the implants of the present invention may be tailor-made to the patient and the damage to her joint to be repaired. This individually shaped implant can be made by the method described in WO2016/004991, filed by the same applicant and having the same filing date.

The advantages of implants over knee replacement have stimulated a further development of smaller implants that can be implanted with less invasive surgery. In this development there has also been an effort to achieve small joint implants, suitable for repair of a small cartilage injury that have a minimal influence on the surrounding parts of the joint. In the current development, such small implants are designed with an implant body that may be formed as a mushroom cap with a hard surface to face the articulating side of the joint and a bone contacting surface engaging the bone below the damaged part of cartilage. The shape and the curvature of the articulating surface of the implant may be designed to be a reconstitution of the shape and the curvature of the part of the joint when it was undamaged. Such implants are usually designed as mushrooms with an implant body or head and with a peg or a rod projecting from the bone contacting side of the implant body for anchoring the implant into the bone.

WO2007/014164 A2 describes a kit comprising a plurality of small joint implants having different predetermined shapes described as circle, oval, L-shape and triangular shape and tools for placing the implants and a method for placing the implant in a joint, e.g. in the knee or other joints where there is a need for repair of a cartilage and/or bone damage. In this piece of prior art each implant shape has a specific guide tool which corresponds to the shape of the implant.

The cartilage damage is repaired by choosing the most suitable implant from the different shapes mentioned above. The corresponding guide tool is selected and is used for faster reaming of the area where the implant is to be placed. A drill is used for drilling a hole to accept the post extending from the bone contacting side of the implant. In the end, the implant is placed on the area reamed or drilled out for the implant. Although it is the intention that the guide tool shall be used for the preparation of the placement of the implant it is also said that the use of the guide tool is optional.

US2003/0144741 describes an implant having pins or pegs which may be press fit or held in place with the use of bone cement.

### The advantages of the invention

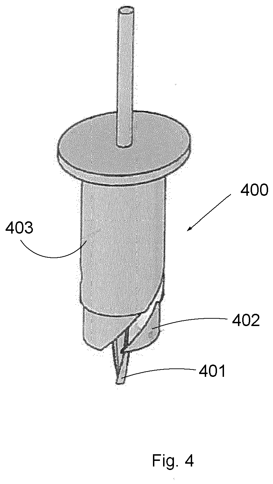

The aim of the present invention is to solve a complex of difficulties encountered when attempting to repair damaged joints using surgical implants. For a number of different types of joint damage, a circular implant mushroom cap with a central anchoring stem or peg of smaller diameter is preferably used. The deeper small diameter central hole for the central anchoring peg and the shallower larger diameter hole for the implant cap having the new joint repair surface can be accurately drilled at the same time where a circular double drill is preferably used. A double drill has a central small diameter bit and further up a larger diameter drill cutting surface. An example of such a drill used together with the drilling rig is shown in Fig. 4. Such drills are commonly used in other areas for inlaid discs and for countersinking screws.

But the area of the joint damage may not be easily covered by a single circular implant if the damaged area is elongate or is irregular or large in shape. Instead of using a number of separate implants or an implant requiring complicated bone removal techniques, using several different drills and tools, the surgical implant and the rig provide an exceptionally simple solution which also utilizes a single rig anchored in place for the entire pre-drilling and drilling operation. The same double-drill, the same pre drilling guide socket and the same depth adjustment socket is used for all drillings. This is made possible by a rig which permits shifting of the guide socket or adjustment socket from one side to the other side (or the other sides) of the hollow shell interior between drillings. A shiftable interior arcuate wall can also be inserted in each position to provide a complete circular cylinder for holding the pre-drilling guide socket for each drilling.

According to one embodiment, this will simply create two identical peg holes and an exactly excavated cavity to fit an implant in the form of two intersecting circles of the same diameter. Merely removing the insert wall in the cylindrical interior then creates a shell, already securely rigged in location, for a gauge for the oblong implant with at least two pegs. A handled gauge in the shape of the implant is inserted after drilling to check that the proper drilling depth has been reached. After all drillings have been made and depth checked, the drilling rig is removed.

The implant should comprise a biocompatible metal, metal alloy, ceramic or polymeric material. More specifically it may comprise any metal or metal alloy used for structural applications in the human or animal body, such as stainless steel, cobalt-based alloys, chrome-based alloys, titanium-based alloys, pure titanium, zirconium-based alloys, tantalum, niobium and precious metals and their alloys. If a ceramic is used as the biocompatible material, it can be a biocompatible ceramic such as aluminium oxide, silicon nitride or yttria-stabilized zirconia. Preferably the articulate surface comprises a cobalt chrome alloy (CoCr) or stainless steel, diamond-like carbon or a ceramic.

The implant according to one embodiment of the present invention has two parallel pegs of the same nominal diameter, but with one being slightly larger than the diameter of the hole to provide an interference fit. The other peg of the same nominal diameter is very slightly smaller than the diameter of the hole to provide a slide fit. This relationship will provide secure anchoring of the implant by virtue of the interference fit. The slide fit peg will prevent rotation of the implant and will not give rise to problematic stresses between the pegs which might be the case with two interference fits. This implant and rig will also make is easier to insert and make sure that the implant cap seats securely in place against the bottom of the shallow wide hole drilled into the bone. This is very important in making sure that the implant is held securely by bone growth without cavities.

The present invention also contemplates as a first alternative a surgical implant having two pegs, but it is also contemplated according to the invention an implant having three or more pegs and an implant form comprising three or more intersecting circles. In this case the drill guide insert wall is shifted between three or more different arcuate depressions in the interior of the rig.

### Short Description of the Drawings

The implant and rig will be described below with reference to a non-limiting example shown in the accompanying drawings of which,
Fig. 1 shows a two-pegged implant,
Fig.2 shows a rig for a two pegged implant. The rig is mounted in place on a femoral condyle.
Fig. 3a shows a three-pegged implant having the form of three identical intersecting circles.
Fig.3b shows from above a rig with wall insert for a three-peg implant.
Fig. 4 shows a double drill for use with the drilling rig.
Fig. 5a shows a pre-drilling guide socket.
Fig. 5b shows a drilling depth adjustment socket.

### Detailed Description

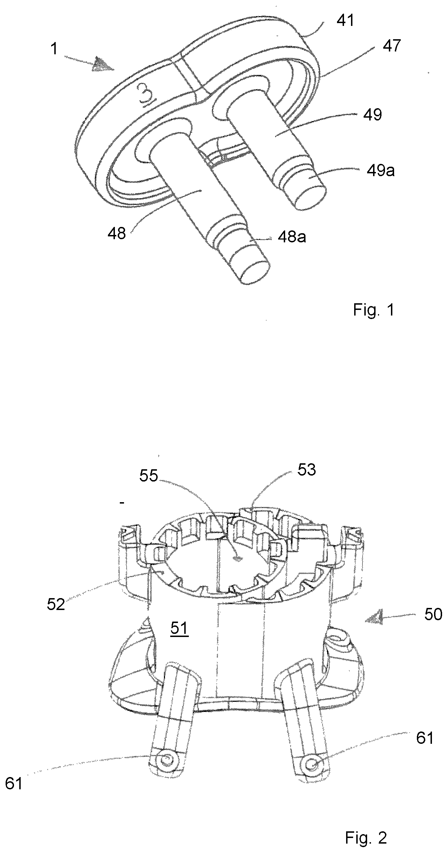

Fig. 1 shows one exemplary implant according to the present invention, in this case for use in the repair of a damaged condylar surface of the human femur. It is contemplated that in certain applications of the invention the outer surface of cap 3 of the implant 1 will be shaped to conform to the undamaged shape of the patient's condyle. Standard sized and shaped implants will also be covered by the scope of the main claim. Such implants can also be used for many different joint surfaces in, for example, the joints of the hip, knee, toe and shoulder.

The implant 1 has a cap 3 with on its outside 41 a new joint surface and on its inside, in this particular embodiment, a ridge 47 which lodge in a drilled groove as will be explained below. The implant cap has the shape of two intersecting circles of the same diameter. Typical implants according to the invention may have a cap with two intersecting circles of diameter 15 mm. Other shapes which may be suitable are 17+17 mm, 20+20mm and 25+25 mm. At the center of each circle there extends a peg 48, 49. Each peg has, in this particular embodiment, a narrower end 48a, 49a to aid in directing the pegs correctly into drilled holes in the condyle, as will be explained in more detail below.

In this case, the first peg 48 is longer than the second peg 49, but they can also be of the same length. According to the invention, both pegs are of the same nominal diameter, but the first peg 48 is slightly larger than the nominal diameter, providing an interference fit shaft of said nominal diameter. An anchoring interference fit between hard metal and living bone requires a greater differential than an interference fit between two metal elements. How much larger than the nominal diameter the first peg is will be a matter of clinical testing and revision. In this context involving a metal shaft in a hole in living bone and in the appended claims the term interference fit in relation to a nominal hole diameter is deemed to include positive differences up to and including approximately +11% increase in diameter over the nominal diameter. To get a very secure grip between a hole of a diameter of 4 mm in living bone and a peg of one of the materials described in the paragraph above, the peg should have a diameter of between ca 4.1 and 4.4 mm. An interference fit between hard metal and living bone requires a significantly larger difference than between a shaft and a hole of hard metal for example. The differential between the first peg diameter and the hole should not be so great as to require excessive force to put it in place with the risk of cracking in the bone. The second peg 49 has a diameter of the same nominal diameter but falling within the standard definitional boundaries for a clearance fit, i.e. almost of the same diameter but very slightly smaller. This relationship will ensure that the implant is securely anchored, is fairly easy to install, and will not give rise to problematic stresses between the pegs, either during implantation or thereafter.

Fig. 2 shows an example of a rig which is used for all of the hole preparation. The rig comprises an elongated hollow shell 51 having the form of two intersecting (overlapping) right circular cylinders 52, 53 of the same diameter. The rig can be formed to conform to the shape of the bone and cartilage area of the patient to be repaired or can be a standard rig. The rig is held securely in place on the condylar surface in this case by pins (not shown) driven in through holes 61, to hold the rig securely in place throughout the entire drilling process.

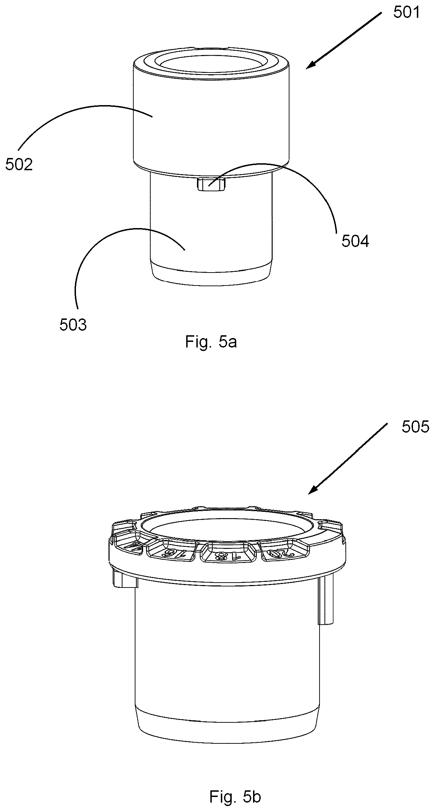

After the pins have been driven in, the cutting and drilling process can begin, with a wall insert 55 inserted in one end of the hollow shell, leaving an entire first right circular cylinder 52 at one end of the hollow tubular shell. At this time the surgeon may insert into the first right circular cylinder a depth adjustment socket 505 (Fig. 5b) and then a sharp cylindrical hand knife, sized exactly to the interior of the adjustment socket 505, make a preliminary circular sharp edged cut through the cartilage down to the bone. A circular bare bone area is left after this cartilage removal.

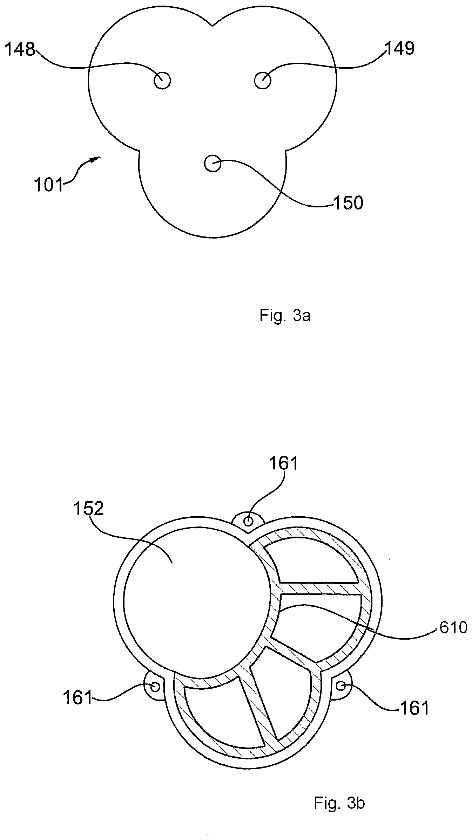

In one embodiment, the surgeon uses a 17/4 mm double drill as shown schematically in Fig. 4. It has a central narrow 4 mm diameter bit 401, and a wider 17 mm diameter cutting bit 402. The outer lateral surface 403 of the double drill conforms to a height adjustment socket placed inside the wall insert, which securely holds the double drill to drill, in the same operation, a central 4 mm hole for the first peg 48 and a much shallower surrounding bore 17 mm in diameter in this example. A pre-drilling of the initial part of the peg hole in the bone can be made using a guide socket 501 (Fig. 5a). This improves the exact placement of the simultaneous drilling of the peg hole and the circular bare-bone area with the double drill (Fig. 3). After removing the drill, and flushing out organic matter, the surgeon then slides the wall insert 53 out and inserts it in on the other side of the hollow shell, creating a complete right circular cylindrical guide hole on the opposite side of the hollow shell.

The surgeon then inserts the adjustment socket and uses the same cylindrical knife in the newly created guide hole, to make a circular excision of the cartilage (not a complete circle since the intersecting portion has already been removed in the previous step). The in this embodiment 17/4 mm double drill is then used again first with the guide socket 501 to pre-drill the peg hole and then with the adjustment socket 505 to double-drill the peg hole to its full depth and create the bare-bone circle, i.e. the 4 mm hole for the second peg and a second surrounding shallow bore which is of course also 17 mm in diameter.

These two drilling operations have created 4 mm peg holes and a space in the bone to exactly accommodate in this case a 17+17 implant of the invention. The wall insert 53 is then completely removed. A handle-equipped gauge corresponding to the intersecting circular forms making up the implant, is used to make sure that the holes have been drilled to the proper depth in the bone. The rig is then removed and the implant pegs are inserted into their holes. For the cap of the implant to lodge exactly in the in this case 17+17 shallow cavity removed from the surface of the bone it is usually necessary to carefully tap the cap, preferably on top of the first peg, with interference fit, with a hammer via a special mandrel. The first, slightly thicker peg, is tapped down into its hole while the second peg, slightly narrower, slides easily into its hole. The larger diameter part of the 17/4 mm drill in this example has a rim to excavate a peripheral slot slightly deeper than the 17 mm shallow cavity, to accommodate the peripheral ridge 47 of the implant, helping to hold the implant securely in place during healing and subsequent loading during use.

Thus the rig, which can be form-fitted to the shape of the individual patient's condyle in this example, is placed over the damaged area of the condyle and is anchored securely in place, in this particular non-limiting example, by driving in four pins (not shown) into holes 61 in the condyle shaped lower end of the rig 50. It is now securely in place for the entire drilling operation, which be simplified greatly and made much more exact and less dependent on the artistry of the surgeon, which may vary from day to day.

After drilling of the holes, the pins are pulled out and the rig is removed from the site, for implantation of the implant and reconstitution of the joint with the new implant.

It will be understood by the person skilled in the art that the rig as claimed can be supplemented with for example an insert sleeve to make one of the right circular cylinders of a small diameter, e.g. from 17 to 15 mm in diameter, to accommodate an implant having the form of two intersecting circles of slightly different diameters, for example 15 + 17 millimeters.

It will of course also be possible, within the scope of the invention to create an implant in the form of three, or more, intersecting circles, to cover bone damage of more irregular shape. One such three-circle implant 101 is shown from below in Fig. 3a showing three pegs 148, 149 and 150. In this example peg 148 has an interference fit diameter in relation to the common nominal diameter of all three pegs and the other two pegs 149 and 150 have clearance fit diameters in relation to the common nominal diameter.

The rig for this three-circle implant is shown from above in Fig. 3b. The rig is held in place on the bone by pins (not shown) inserted through holes 161. The wall insert 155, completes the first right circular cylinder 152 covering the remaining portions of the other two right circular cylinders. When the first circular drilling has been made the wall insert 155 is pulled out, rotated 120 degrees and is inserted again to provide a drill guide for the next circle drilling with the same double drill, which in one embodiment can be the same 17/4 drill used together with the two-circle rig. After rotation 120 degrees again and drilling, a three pegged implant is inserted. As stated above, this insert has one peg which is of interference fit dimension in relation to its nominal diameter (in this case 4 mm) and the other two pegs are of clearance fit.

The implant has a bone contact surface on the underside, on the sides of the cap and on the pegs, which will be in direct contact with the bone tissue when the implant is in place. In one embodiment the bone contact surface comprises a biocompatible metal, metal alloy or ceramic, such as any of the metals, metal alloys or ceramic described above for the articulate surface. Preferably the bone contact surface comprises a cobalt chrome alloy (CoCr), a titanium alloy, titanium or stainless steel.

In one specific non-limiting embodiment the bone contact surface comprises, or in one specific non-limiting embodiment is coated with, a material that promotes osseointegration. In an alternative embodiment the bone contact surface does not comprise such a material and/or is uncoated.

The bioactive material or the material that promotes osseointegration of the bone contact surface, if present, preferably stimulates bone to grow into or onto the implant surface. Several materials that have a stimulating effect on bone growth are known and have been used to promote adherence between implants and bone. Examples of such prior art materials include bioactive glass, bioactive ceramics and biomolecules such as collagens, fibronectin, osteonectin and various growth factors. A commonly used material in the field of implant technology is hydroxyapatite (HA), chemical formula Ca₁₀(PO₄)₆(OH)₂. HA is the major mineral constituent of bone and is able to slowly bond with bone in *vivo.* HA coatings have been developed for medical implants to promote bone attachment. Another bioactive material commonly used in prior art is bioactive glass. Bioactive glasses, generally comprising SiO2, CaSiO₃, P₂O₅, Na₂O and/or CaO and possibly other metal oxides or fluorides, are able to stimulate bone growth faster than HA.

The fixation of the implant can also be improved by decreasing the catabolic processes i.e. decrease the amount of bone resorption next to the implant. The bone contact surface and/or the extending post can also be modified with bisphosphonates.

In one embodiment the bone contact surface is coated with a double coating. Such double coating may for instance comprise an inner coating comprising titanium (Ti). The second, outer coating, that is configured to contact the cartilage and or bone, is preferably a hydroxyapatite and/or beta tricalcium phosphate (TCP) coating. By this design even more long-term fixation of the implant is achieved, since bone in- or on-growth to the implant is further stimulated by the titanium, even if the more brittle hyroxyapatite would eventually shed/dissolve.

The bone contact surface may also be further modified with fluoro compounds or acid etching to enhance the bioactivity and the osseointegration of the surface. Another method to facilitate osseointegration is blasting of the bone contact surface.

Fig. 4 shows an exemplary 4/17 double drill for use with the multiple circle rigs described above (or with a previously known single circle rig). The double drill has a 4 mm central bit 401 for creating the hole for the peg and a wider cutting surface 402 for creating the 17 mm shallow hole. One of the advantages is that the same double drill can be used for single, double or triple (or more) intersecting circle shaped implants, used twice or three times as the case may be for the two embodiments shown here.

## Claims

1. Surgical joint implant having a cap (3) with an articular outer surface and an inner surface adapted for bone adhesion, said cap having at least first (48) and second (49) pegs extending from said inner surface for anchoring said implant in a bone, **characterized in that** said at least first (48) and second (49) pegs have the same nominal diameter and that the diameter of said first peg deviates positively from said nominal diameter to provide an interference fit in relation to said nominal diameter and **in that** said second peg deviates negatively from said same nominal diameter to provide a clearance fit in relation to said same nominal diameter.

2. Surgical joint implant according to Claim 1, **characterized in that** said first peg (48) deviates from said nominal diameter to provide a force fit in relation to said nominal diameter.

3. Surgical joint implant according to Claim 1 or 2, **characterized in that** said second peg (49) deviates from said nominal diameter to provide a slide fit in relation to said nominal diameter.

4. Surgical joint implant according to one of the preceding claims, **characterized by** a third peg (150) which deviates from said nominal diameter to provide a slide fit in relation to said nominal diameter.

5. Surgical joint implant according to Claim 1, **characterized in that** said cap (3) has the form of two intersecting circles of the same diameter, said first and second pegs (48, 49) being disposed at the centers of the respective circles.

6. Surgical joint implant according to Claim 4, **characterized in that** the cap has the form of three intersecting circles of the same diameter, said first, second and third pegs (148),(149), (150) being disposed at the centers of the respective circles.

7. Surgical joint implant according to Claim 1, **characterized in that** one (48) of the pegs is significantly longer than the others.

8. Surgical joint implant according to one of the preceding claims, **characterized in that** said articular outer surface is individually custom-formed.

9. Surgical joint implant according to one of the preceding claims, **characterized in that** said inner surface adapted for bone adhesion and the surfaces of said pegs have an outer layer of a substance that promotes bone growth.

## Patentansprüche

1. Chirurgisches Gelenkimplantat mit einer Kappe (3) mit einer Gelenkaußenfläche und einer Gelenkinnenfläche, die für eine Knochenverwachsung ausgelegt ist, wobei die Kappe zumindest erste (48) und zweite (49) Stifte aufweist, die sich von der Innenfläche erstrecken, um das Implantat in einem Knochen zu verankern, **dadurch gekennzeichnet, dass** die zumindest ersten (48) und zweiten (49) Stifte den gleichen Nenndurchmesser aufweisen und dass der Durchmesser des ersten Stifts vom Nenndurchmesser positiv abweicht, um eine Presspassung in Bezug auf den Nenndurchmesser bereitzustellen, und dass der zweite Stift vom gleichen Nenndurchmesser negativ abweicht, um eine Spielpassung in Bezug auf den gleichen Nenndurchmesser bereitzustellen.

2. Chirurgisches Gelenkimplantat nach Anspruch 1, **dadurch gekennzeichnet, dass** der erste Stift (48) vom Nenndurchmesser abweicht, um einen Festsitz in Bezug auf den Nenndurchmesser bereitzustellen.

3. Chirurgisches Gelenkimplantat nach Anspruch 1 oder 2, **dadurch gekennzeichnet, dass** der zweite Stift (49) vom Nenndurchmesser abweicht, um einen Gleitsitz in Bezug auf den Nenndurchmesser bereitzustellen.

4. Chirurgisches Gelenkimplantat nach einem der vorstehenden Ansprüche, **gekennzeichnet durch** einen dritten Stift (150), der vom Nenndurchmesser abweicht, um einen Gleitsitz in Bezug auf den Nenndurchmesser bereitzustellen.

5. Chirurgisches Gelenkimplantat nach Anspruch 1, **dadurch gekennzeichnet, dass** die Kappe (3) die Form von zwei sich schneidenden Kreisen mit dem gleichen Durchmesser aufweist, wobei die ersten und zweiten Stifte (48, 49) in den Mitten der jeweiligen Kreise angeordnet sind.

6. Chirurgisches Gelenkimplantat nach Anspruch 4, **dadurch gekennzeichnet, dass** die Kappe die Form von drei sich schneidenden Kreisen mit dem gleichen Durchmesser aufweist, wobei die ersten, zweiten und dritten Stifte (148), (149), (150) in den Mitten der jeweiligen Kreise angeordnet sind.

7. Chirurgisches Gelenkimplantat nach Anspruch 1, **dadurch gekennzeichnet, dass** der eine (48) der Stifte signifikant länger als die anderen ist.

8. Chirurgisches Gelenkimplantat nach einem der vorstehenden Ansprüche, **dadurch gekennzeichnet, dass** die Gelenkaußenfläche individuell nach Maß gebildet wird.

9. Chirurgisches Gelenkimplantat nach einem der vorstehenden Ansprüche, **dadurch gekennzeichnet, dass** die Innenfläche, die für eine Knochenverwachsung ausgelegt ist, und die Oberflächen der Stifte eine Außenschicht einer knochenwachstumsfördernden Substanz aufweisen.

## Revendications

1. Implant d'articulation chirurgical ayant une coiffe (3) avec une surface externe articulaire et une surface interne adaptée pour une adhésion à un os, ladite coiffe ayant au moins des premier (48) et deuxième (49) plots s'étendant depuis ladite surface interne pour ancrer ledit implant dans un os, **caractérisé en ce que** lesdits au moins premier (48) et deuxième (49) plots ont le même diamètre nominal et **en ce que** le diamètre dudit premier plot s'écarte positivement dudit diamètre nominal pour fournir une mise en place avec ajustement serré par rapport audit diamètre nominal et **en ce que** ledit deuxième plot s'écarte négativement dudit même diamètre nominal pour fournir une mise en place avec jeu par rapport audit même diamètre nominal.

2. Implant d'articulation chirurgical selon la revendication 1, **caractérisé en ce que** ledit premier plot (48) s'écarte dudit diamètre nominal pour fournir une mise en place forcée par rapport audit diamètre nominal.

3. Implant d'articulation chirurgical selon la revendication 1 ou 2, **caractérisé en ce que** ledit deuxième plot (49) s'écarte dudit diamètre nominal pour fournir une mise en place avec glissement par rapport audit diamètre nominal.

4. Implant d'articulation chirurgical selon l'une des revendications précédentes, **caractérisé par** un troisième plot (150) qui s'écarte dudit diamètre nominal pour fournir une mise en place avec glissement par rapport audit diamètre nominal.

5. Implant d'articulation chirurgical selon la revendication 1, **caractérisé en ce que** ladite coiffe (3) a la forme de deux cercles en intersection de même diamètre, lesdits premier et deuxième plots (48, 49) étant disposés aux centres des cercles respectifs.

6. Implant d'articulation chirurgical selon la revendication 4, **caractérisé en ce que** la coiffe a la forme de trois cercles en intersection de même diamètre, lesdits premier, deuxième et troisième plots (148), (149), (150) étant disposés aux centres des cercles respectifs.

7. Implant d'articulation chirurgical selon la revendication 1, **caractérisé en ce que** l'un (48) des plots est sensiblement plus long que les autres.

8. Implant d'articulation chirurgical selon l'une des revendications précédentes, **caractérisé en ce que** ladite surface externe articulaire est formée individuellement, sur mesure.

9. Implant d'articulation chirurgical selon l'une des revendications précédentes, **caractérisé en ce que** ladite surface interne adaptée pour une adhésion à un os et les surfaces desdits plots ont une couche externe d'une substance qui favorise la croissance osseuse.
